# EUROPEAN PATENT SPECIFICATION

(11) **EP 1 994 018 B1**
(45) Date of publication and mention of the grant of the patent: **28.11.2012**
(21) Application number: 07726784.7
(22) Date of filing: 12.03.2007
(51) Int. Cl.: C07D 317/36, C07C 68/04, C07C 69/96, C07C 31/18, C07C 29/12, C07C 29/128, C07C 68/06

(54) **PROCESS FOR THE PRODUCTION OF ALKYLENE CARBONATE AND USE OF ALKYLENE CARBONATE THUS PRODUCED IN THE MANUFACTURE OF AN ALKANE DIOL AND A DIALKYL CARBONATE**
VERFAHREN ZUR HERSTELLUNG VON ALKYLENCARBONAT UND VERWENDUNG VON IN DIESEM VERFAHREN HERGESTELLTEM ALKYLENCARBONAT ZUR HERSTELLUNG EINES ALKANDIOLS UND EINES DIALKYLCARBONATS
PROCÉDÉ DE PRODUCTION DE CARBONATE D'ALKYLÈNE ET UTILISATION DU CARBONATE D'ALKYLÈNE AINSI PRODUIT DANS LA FABRICATION D'UN ALCANEDIOL ET D'UN CARBONATE DE DIALKYLE

(30) Priority: 13.03.2006 EP 06110999
(43) Date of publication of application: 26.11.2008
(73) Proprietor: Shell Internationale Research Maatschappij B.V., 2596 HR Den Haag (NL)
(72) Inventor: VAN DER HEIDE, Evert, NL-1031 CM Amsterdam (NL); VAN KESSEL, Gerardus Martinus Maris, NL-1031 CM Amsterdam (NL); NISBET, Timothy Michael, NL-1031 CM Amsterdam (NL)
(74) Representative: Matthezing, Robert Maarten
(86) International application number: PCT/EP2007/052270
(87) International publication number: WO 2007/104730

(56) References cited:
- WO-A-2005/003113
- PEPPEL W J: "Preparation and Properties of the Alkylene Carbonates" INDUSTRIAL AND ENGINEERING CHEMISTRY, AMERICAN CHEMICAL SOCIETY, US, vol. 50, no. 5, May 1958 (1958-05), pages 767-770, XP002396365

## Description

The present invention relates to a process for the production of alkylene carbonate and the use of alkylene carbonate thus produced in the manufacture of an alkane diol and a dialkyl carbonate.

Processes for the production of alkylene carbonates are known. WO-A 2005/003113 discloses a process in which carbon dioxide is contacted with an alkylene oxide in the presence of a suitable catalyst. The catalyst disclosed is a tetraalkyl phosphonium compound: This specification discloses that the catalyst used has been recycled. The specification further discloses that the performance of the catalyst is very stable if the catalyst is recycled to the alkylene carbonate preparation in an alcohol, in particular in propylene glycol (1,2-propane diol).

US-A 4,434,105 also discloses a process for the preparation of alkylene carbonates. Various catalysts are disclosed. The document also describes that the catalyst after completion of the reaction may be reused.

In a continuous process the reaction product containing alkylene carbonate and catalyst has to be subjected to a work-up treatment. Such work-up treatment generally includes one or more distillation steps to separate the product from the catalyst. It has been found that the catalyst activity decreases if the catalyst is being reused without taking appropriate steps to remove contaminants in the catalyst to be recycled. These contaminants include decomposition products of the phosphonium catalyst. None of the above-mentioned documents provide a method of avoiding a build-up of any such contaminants.

It has now been found that catalyst activity can be retained by purifying at least part of the catalyst from the product.

Accordingly, the present invention provides a process for the production of an alkylene carbonate by the reaction of an alkylene oxide with carbon dioxide in the presence of a phosphonium catalyst in which process
(a) the alkylene oxide, carbon dioxide and the phosphonium catalyst are continuously introduced into a reaction zone from which a product stream containing alkylene carbonate and used phosphonium catalyst is withdrawn,
(b) alkylene carbonate and a stream containing used phosphonium catalyst are separated from the product stream,
(c) the alkylene carbonate, separated in step (b), is recovered as product,
(d) at least a part of the stream containing used phosphonium catalyst is subjected to purification, to obtain purified phosphonium catalyst, and
(e) purified phosphonium catalyst is recycled to the reaction zone.

The process according to the present invention allows that the catalyst can be used for a long period in a continuous process. It has been found that the reason therefore may be related to the formation of decomposition products in the catalyst during the preparation of alkylene carbonate. It has been found that contaminants of the phosphonium catalyst include phosphine oxides. By purification of the catalyst used, the phosphine oxides can effectively be removed so that active catalyst can be recycled to the reaction zone in step (a). A further advantage of the present process resides in the fact that the process pre-empts the necessity to include a bleed stream via which contaminated catalyst has to be withdrawn from the process.

The catalyst is a phosphonium compound. Such catalysts are known, e.g., from US-A 5,153,333, US-A 2,994,705, US-A 4,434,105, WO-A 99/57108, EP-A 776,890 and WO-A 2005/003113. Preferably, the catalyst is a phosphonium halide of formula R₄PHal, in which Hal means halide and each R can be the same or different and can be selected from an alkyl, alkenyl, cyclic aliphatic or an aromatic group. The group R suitably contains from 1 to 12 carbon atoms. Good results are obtained with R being a C₁₋₈ alkyl group. Most preferred are groups R being selected from methyl, ethyl, n-propyl, isopropyl, n-butyl, isobutyl, and t-butyl groups. Preferably, the halide ion is bromide or iodide. It appeared that the bromide and iodide compounds are more stable than the corresponding chloride compounds. The most preferred phosphonium catalyst is tetra (n-butyl) phosphonium bromide. An additional advantage of the present process resides in that there is not the need to the costly treatment of a halogen-containing catalyst bleed stream. The purification of the used phosphonium catalyst may be achieved in various ways. It is possible to subject the used catalyst to extraction, crystallisation adsorption or other separation techniques. It is preferred to subject the used catalyst to distillation.

It has been found that the used catalyst tends to form decomposition products when it is exposed to relatively high temperatures for a prolonged period. It is, therefore, preferred to conduct the distillation at relatively low temperatures. Thereto the distillation is suitably conducted at sub-atmospheric pressures. By using the sub-atmospheric pressure contaminants in the catalyst composition are distilled leaving the purified phosphonium catalyst as distillation residue. The distillation temperature preferably does not exceed 250 °C. More preferably, the distillation temperature ranges from 50 to 200 °C, most preferably from 100 to 180 °C. Suitable pressures for such distillation temperatures are from 0.1 to 0.0001 bar (10 to 0.01 kPa). Preferably the pressure ranges from 0.05 to 0.0005 bar (5,000 to 5 Pa).

It is surprising that these phosphonium catalysts show complete recovery of their activity even when they have been subjected to these distillation conditions.

The phosphonium catalyst tends to be a solid material. The catalyst may be recycled to the reaction zone as a solid. It is also possible to convert the catalyst to a melt and recycle the molten catalyst to the reaction zone. However, since the presence of a solvent shows a stabilising effect on the catalyst it is preferred to recycle the purified phosphonium catalyst to the reaction zone in the presence of a solvent. The solvent can be a carbonyl-containing compound, especially aldehydes, as disclosed in WO-A 2005/051939. More preferably, the solvent is an alcohol. Many alcohols may be selected to increase the stability of the phosphonium catalyst. The alcohol may be monovalent, bivalent, or multivalent. The alcohol may comprise an aliphatic C₁₋₁₂ chain substituted by one or more hydroxyl groups. Aromatic alcohols or alkylaromatic alcohols may also be used, suitably having 6 to 12 carbon atoms. Polyalkylene glycols or the monoalkyl ethers thereof may also be used. Mixtures may also be used.

Preferably, the alcohols used are selected from the group consisting of C₁₋₆ mono-alkanols, C₂₋₆ alkane diols, C₃₋₆ alkane polyols, including glycerol, phenol, C₁₋₆ alkyl substituted phenols, C₆₋₁₂ cycloaliphatic alcohols and mixtures thereof. Very suitable are C₂₋₆ alkane polyols, in particular 1,2-ethane diol, 1,2-propane diol, sorbitol and mixtures thereof. The use of ethane or propane diol has a further advantage when the alkylene carbonate is converted to alkylene glycol (alkane diol), and the alkalene glycol is used as solvent for the phosphonium catalyst. Sorbitol is providing excellent stability to the phosphonium catalyst. It may be advantageous to use a combination of 1,2-ethane or propane diol and sorbitol.

In order to replenish any decomposed catalyst it is effective to add make-up phosphonium catalyst to the reaction zone. The make-up phosphonium catalyst can be added at any place in the process where catalyst is present. Suitably any make-up phosphonium catalyst is added to the process via direct addition to the reaction zone or via addition to the stream of purified phosphonium catalyst that is to be recycled.

In the present process at least part of the stream containing used phosphonium catalyst is subjected to the purification step. It is possible to subject the complete stream and thus all catalyst to this purification. It is, however, preferred to subject only part thereof. By doing so the build up of contaminants is avoided. Moreover, it has been shown that if the phosphonium catalyst contains a minor amount of such contaminants such contaminants do not have a detrimental effect on the catalyst activity. It will be evident that the fact that not all catalyst has to be purified continuously provides a significant economic advantage. Suitably, from 1 to 90 %wt, more preferably from 2% to 50 %wt, most preferably from 5 to 25 %wt of the stream containing used phosphonium catalyst is subjected to purification. It is preferred that also another part of the stream containing used phosphonium catalyst is recycled to the reaction zone, together with purified phosphonium catalyst. More preferably, all of the remaining part of this stream is recycled to the reaction zone. Although there may be no need for a bleed stream, it is possible to apply a minor bleed stream.

The stream containing used phosphonium catalyst suitably contains some alkylene carbonate. The alkylene carbonate ensures that the used phosphonium catalyst is in liquid form, which facilitates transportation, e.g., recycle. Further, it has been found that the combination of alcohol and alkylene carbonate has a stabilising effect on the catalyst. Therefore, if only part of the used phosphonium catalyst is subjected to purification the remaining part of the used catalyst, suitably in combination with alkylene carbonate is recycled to the reaction zone. If the purified phosphonium catalyst has been dissolved in an alcohol, these streams can suitably be combined such that a mixture of used phosphonium catalyst, purified phosphonium catalyst, alcohol and alkylene carbonate is recycled to the reaction zone. If alkylene carbonate is present in the stream containing used phosphonium catalyst the alkylene carbonate is separated from any phosphorus-containing contaminants and catalyst in the purification step. This can be achieved in a distillation column where different fractions are obtained at different trays. However, it may also be achieved in two dedicated steps, wherein in the first step alkylene carbonate is separated from the catalyst and any heavy contaminants, and subsequently, the contaminants are separated from the catalyst to yield the purified phosphonium catalyst. The latter manner has the advantage that optimal distillation conditions may be applied for each separation.

The alkylene oxide that is converted in the present process is suitably a C₂₋₄ alkylene oxide, in particular ethylene oxide or propylene oxide or mixtures thereof.

The amount of phosphonium catalyst in the reaction zone may conveniently be expressed in mole catalyst per mole alkylene oxide. Due to a lower amount of byproducts, the subject process is suitably carried out in the presence of at least 0.0001 mole of the phosphonium catalyst per mole alkylene oxide. Preferably, the amount of phosphonium catalyst present is such that it ranges from 0.0001 to 0.1 mole phosphonium catalyst, more preferably from 0.001 to 0.05, and most preferably from 0.003 to 0.03 mole phosphonium catalyst per mole propylene oxide.

The reaction of carbon dioxide with the alkylene oxide is reversible. That means that the alkylene carbonate formed may convert back into carbon dioxide and the alkylene oxide. The molar ratio between carbon dioxide and alkylene oxide may be as low as 0.5:1, more suitably from 0.75:1. In view of the reversibility of the reaction it is preferred to ensure at least a slight excess of carbon dioxide, such as 1.0:1 to 10:1, more preferably from 1.01:1 to 2:1, most preferably from 1.01:1 to 1.2:1. A suitable means to establish an excess of carbon dioxide is to conduct the reaction at an elevated carbon dioxide pressure and keeping the pressure constant by dosing carbon dioxide. The total pressure ranges suitably from 5 to 200 bar; the partial carbon dioxide partial pressure is preferably in the range from 5 to 70, more preferably from 7 to 50, and most preferably from 10 to 30 bar.

The reaction temperature can be selected from a wide range. Suitably the temperature is selected from 30 to 300 °C. The advantage of relatively high temperature is the increase in reaction rate. However, if the reaction temperature is too high, side reactions, i.a. the degradation of alkylene carbonate to carbon dioxide and propionaldehyde or acetone, the undesired reaction of alkylene oxide with any alkane diol, if present, may occur, or the undesired decomposition of the phosphonium catalyst may be accelerated. Therefore, the temperature is suitably selected from 100 to 220 °C.

The skilled person will be able to adapt other reaction conditions as appropriate. The residence time of the alkylene oxide and the carbon dioxide in the reaction zone can be selected without undue burden. The residence time can usually be varied between 5 min and 24 hours, preferably between 10 minutes and 10 hours. Conversion of alkylene oxide is suitably at least 95%, more preferably at least 98%. Dependent on the temperature and pressure the residence time may be adapted. The catalyst concentration may also vary between wide ranges. Suitable concentrations include from 1 to 25 %wt, based on the total reaction mixture. Good results can be obtained with a catalyst concentration of 2 to 8 %wt, based on the total reaction mixture.

As to the relative amounts of alkylene carbonate and alcohol the skilled artisan can vary the ratio in broad ranges. Very good results have been obtained employing a weight ratio of alkylene carbonate to alcohol of 0.1-10, in particular from 0.2 to 5, more preferably from 0.5 to 2. In view of the chance for the undesired reaction between the alkylene oxide and an alcohol in the reaction zone the amount of alcohol is suitably kept at a relatively low level, such as from 1 to 25 %wt, based on the weight of alkylene oxide, carbon dioxide, alkylene carbonate and alcohol in the reaction zone. Preferably the amount of alcohol ranges from 5 to 20 %wt.

The alkylene carbonate that is produced in the present process can suitably be used for the production of alkane diol and dialkylcarbonate. Accordingly, the present invention also provides a process for the preparation of alkane diol and dialkyl carbonate comprising reacting an alkanol and alkylene carbonate over a transesterification catalyst in which the alkylene carbonate has been prepared by the process of the present invention, and recovering the alkane diol and the dialkyl carbonate from the resulting reaction mixture. The alkanol is suitably a C₁₋₄ alcohol. Preferably the alkanol is methanol, ethanol or isopropanol.

The transesterification reaction in itself is known. In this context reference is made to US-A 4,691,041, disclosing a process for the manufacture of ethylene glycol and dimethyl carbonate by the transesterification reaction over a heterogeneous catalyst system, in particular an ion exchange resin with tertiary amine, quaternary ammonium, sulphonic acid and carboxylic acid functional groups, alkali and alkaline earth silicates impregnated into silica and ammonium exchanged zeolites. US-A 5,359,118 and US-A 5,231,212 disclose a continuous process for preparing dialkyl carbonates over a range of catalysts, including alkali metal compounds, in particular alkali metal hydroxides or alcoholates, such as sodium hydroxide or methanolate, thallium compounds, nitrogen-containing bases such as trialkyl amines, phosphines, stibines, arsenines, sulphur or selenium compounds and tin, titanium or zirconium salts. According to WO-A 2005/003113 the reaction of alkylene carbonate with an alkanol is conducted over heterogeneous catalysts, e.g. alumina. In this document it is proposed to remove the phosphonium catalyst together with the alkane diol, i.e., after the conversion of alkylene carbonate to alkane diol. However, according to the present invention it is preferred to separate the alcohol, if present, at an earlier stage. According to the present invention the alcohol is preferably separated from the product stream containing alkylene carbonate and used phosphonium catalyst. In this way the amount of alcohol to be recycled can be kept to a minimum. Moreover, any light halide compound that may be formed during the reaction as by-product is removed from the alkylene carbonate product and cannot hinder any subsequent process step. It is preferred to use the alkane diol as the solvent in the presence of which purified phosphonium catalyst is recycled to the reaction zone in which carbon dioxide and alkylene oxide are reacted to yield alkylene carbonate. In this way the presence of extraneous alcohols is avoided.

In accordance with the above the present invention further provides a process for the production of an alkylene carbonate by the reaction of an alkylene oxide with carbon dioxide in the presence of a phosphonium catalyst in which process
(a) the alkylene oxide, carbon dioxide and the phosphonium catalyst are continuously introduced into a reaction zone from which a product stream containing alkylene carbonate and used phosphonium catalyst is withdrawn, and
(b) alkylene carbonate and a stream containing used phosphonium catalyst are separated from the product stream. The separation can suitably be achieved via distillation. The alkylene carbonate product is usually, optionally after separation from a lighter alcohol, recovered as top product. The bottom product contains the used phosphonium catalyst and some alkylene carbonate. A part of this bottom stream is then subjected to purification via a separate distillation section in accordance with the process of the present invention. The purified phosphonium catalyst thus obtained is suitably dissolved in alkane diol and the solution is combined with a remaining part of the stream containing the used phosphonium catalyst and alkylene carbonate. The obtained combination of alkylene carbonate, alcohol, used phosphonium catalyst and purified phosphonium catalyst is recycled to the reaction zone.

The invention will be further elucidated by means of the following examples.

### EXAMPLES

### EXAMPLE 1

To show that the purification of used phosphonium catalyst can be achieved the following experiment was conducted.

Used catalyst solution (100 ml), comprising about 75 %wt of propylene carbonate and 25 %wt of used phosphonium catalyst composition was subjected to distillation in a glass round-bottom bottle. The used catalyst composition contained 18.2 mole% of tributyl phosphine oxide, the remainder being tetrabutyl phosphonium bromide. A first fraction was removed by distillation under vacuum at 65 °C and 2 mbar (200 Pa). This fraction consisted mainly of propylene carbonate. The residue solidified at cooling and was melted again at heating. The melt was subjected to distillation at 160 °C and 1 mbar (100 Pa). A second fraction was recovered, consisting mainly of tributyl phoshine oxide. The residue that remained in the bottle solidified and consisted mainly of tetrabutyl phosphonium bromide. Analysis showed that the residue contained 1.7 mol% of tributyl phosphine oxide.

### EXAMPLE 2

To show that the purified phosphonium catalyst has regained its catalytic activity two experiments were conducted. In both experiments 120 g propylene oxide were introduced into a 1-litre autoclave. The autoclave was pressurised with CO₂ and heated to 150 °C. Additional CO₂ was introduced till a pressure of 20 bar was reached. A solution of 250 mg of phosphonium bromide catalyst in 5 g 1,2-propanediol was introduced into the autoclave. 10 g of additional 1,2-propane diol was introduced. The pressure was kept constant at 20 bar by dosing CO₂ into the autoclave. After five hours CO₂ introduction was stopped and the autoclave was allowed to cool down. The amount of propylene carbonate, the conversion rate and the selectivity were determined for the two experiments.

The experiments were conducted in the same manner, the only difference being that in the case of experiment 1 the catalyst was taken from the residue of Example 1, and that in the case of experiment 2 fresh high purity tetrabutyl phosphonium bromide (ex Fluka) was applied. The results are shown in the table below.

**Table**

| Experiment No. | Propylene Carbonate, g | Conversion, % | Selectivity, % |
|---|---|---|---|
| 1 | 187,8 | 90.7 | 99.7 |
| 2 | 190.5 | 90.6 | 99.8 |

## Claims

1. Process for the production of an alkylene carbonate by the reaction of an alkylene oxide with carbon dioxide in the presence of a phosphonium catalyst in which process
(a) the alkylene oxide, carbon dioxide and the phosphonium catalyst are continuously introduced into a reaction zone from which a product stream containing alkylene carbonate and used phosphonium catalyst is withdrawn,
(b) alkylene carbonate and a stream containing used phosphonium catalyst are separated from the product stream,
(c) the alkylene carbonate, separated in step (b), is recovered as product,
(d) at least a part of the stream containing used phosphonium catalyst is subjected to purification, to obtain purified phosphonium catalyst, wherein the part of the stream containing used phosphonium catalyst that is purified, is subjected to distillation at a distillation temperature ranging from 50 to 200 °C and a pressure for the distillation from 0.1 to 0.0001 bar (10 to 0.01 kPa), and
(e) purified phosphonium catalyst is recycled to the reaction zone.

2. Process according to claim 1, wherein the catalyst is a phosphonium halide of formula R₄PHal, in which Hal means halide and each R can be the same or different and can be selected from an alkyl, alkenyl, cyclic aliphatic or an aromatic group

3. Process according to claim 2, wherein the catalyst is tetra (n-butyl) phosphonium bromide.

4. Process according to any one of claims 1 to 3, wherein the distillation temperature ranges from 100 to 180 °C.

5. Process according to any one of claims 1 to 4, wherein the purified phosphonium catalyst is recycled to the reaction zone in the presence of a solvent.

6. Process according to any one of claims 1 to 5, wherein from 1 to 90 %wt, more preferably from 2 to 50 %wt, most preferably from 5 to 25 %wt of the stream containing used phosphonium catalyst is subjected to purification.

7. Process according to any one of claims 1 to 6, wherein another part of the stream containing used phosphonium catalyst is recycled to the reaction zone.

8. Process according to any one of claims 1 to 7, wherein a mixture of used phosphonium catalyst, purified phosphonium catalyst, alcohol and alkylene carbonate is recycled to the reaction zone.

9. Process for the preparation of alkane diol and dialkyl carbonate comprising preparing an alkylene carbonate by the reaction of an alkylene oxide with carbon dioxide in the presence of a phosphonium catalyst by the process according to any one of claims 1 to 8, reacting an alkanol and the alkylene carbonate over a transesterification catalyst, and recovering the alkane diol and the dialkyl carbonate from the resulting reaction mixture.

10. Process according to claim 9, wherein the alkane diol is used as the solvent in the presence of which purified phosphonium catalyst is recycled to the reaction zone.

## Patentansprüche

1. Verfahren zur Herstellung eines Alkylencarbonats durch die Reaktion eines Alkylenoxids mit Kohlendioxid in Gegenwart eines Phosphoniumkatalysators, in welchem Verfahren
(a) das Alkylenoxid, Kohlendioxid und der Phosphonium Katalysator kontinuierlich in eine Reaktionszone eingebracht werden, aus welcher ein Alkylencarbonat und gebrauchten Phosphoniumkatalysator enthaltender Produktstrom entnommen wird,
(b) Alkylencarbonat und ein gebrauchten Phosphoniumkatalysator enthaltender Strom aus dem Produktstrom abgetrennt werden,
(c) das Alkylencarbonat, welches im Schritt (b) abgetrennt wird, als Produkt gewonnen wird,
(d) wenigstens ein Teil des gebrauchten Phosphoniumkatalysator enthaltenden Stroms einer Reinigung unterworfen wird, um gereinigten Phosphoniumkatalysator zu erhalten, wobei der Teil des gebrauchten Phosphoniumkatalysator enthaltenden Stroms, welcher gereinigt wird, einer Destillation bei einer Destillationstemperatur im Bereich von 50 bis 200°C und einem Druck für die Destillation von 0,1 bis 0,0001 bar (10 bis 0,01 kPa) unterworfen wird,
(e) gereinigter Phosphoniumkatalysator zur Reaktionszone zurückgeführt wird.

2. Verfahren nach Anspruch 1, wobei der Katalysator ein Phosphoniumhalogenid der Formel R₄PHal ist, worin Hal Halogenid bedeutet und jedes R gleich oder verschieden sein kann und unter einer Alkyl-, Alkenyl-, zyklischen aliphatischen oder einer aromatischen Gruppe ausgewählt sein kann.

3. Verfahren nach Anspruch 2, wobei der Katalysator Tetra-n-butylphosphoniumbromid ist.

4. Verfahren nach einem der Ansprüche 1 bis 3, wobei die Destillationstemperatur von 100 bis 180°C reicht.

5. Verfahren nach einem der Ansprüche 1 bis 4, wobei der gereinigte Phosphonium Katalysator zur Reaktionszone in Gegenwart eines Lösungsmittels zurückgeführt wird.

6. Verfahren nach einem der Ansprüche 1 bis 5, wobei 1 bis 90 Gew.-%, stärker bevorzugt 2 bis 50 Gew.-%, am stärksten bevorzugt 5 bis 25 Gew.-% des gebrauchten Phosphoniumkatalysator enthaltenden Stroms der Reinigung unterzogen werden.

7. Verfahren nach einem der Ansprüche 1 bis 6, wobei ein weiterer Teil des Stroms, enthaltend gebrauchten Phosphoniumkatalysator, in die Reaktionszone zurückgeführt wird.

8. Verfahren nach einem der Ansprüche 1 bis 7, wobei eine Mischung aus gebrauchtem Phosphoniumkatalysator, gereinigtem Phosphoniumkatalysator, Alkohol und Alkylencarbonat in die Reaktionszone zurückgeführt wird.

9. Verfahren zur Herstellung von Alkandiol und Dialkylcarbonat, umfassend das Herstellen eines Alkylencarbonats durch die Reaktion eines Alkylenoxids mit Kohlendioxid in Gegenwart eines Phosphoniumkatalysators durch das Verfahren nach einem der Ansprüche 1 bis 8, Umsetzen eines Alkanols und des Alkylencarbonats über einem Umesterungskatalysator und Gewinnen des Alkandiols und des Dialkylcarbonats aus der erhaltenen Reaktionsmischung.

10. Verfahren nach Anspruch 9, wobei das Alkandiol als Lösungsmittel verwendet wird, in dessen Gegenwart gereinigtem Phosphoniumkatalysator in die Reaktionszone zurückgeführt wird.

## Revendications

1. Procédé de production d'un carbonate d'alkylène par la réaction d'un oxyde d'alkylène avec du dioxyde de carbone en présence d'un catalyseur de phosphonium, procédé dans lequel
(a) l'oxyde d'alkylène, le dioxyde de carbone et le catalyseur de phosphonium sont introduits de façon continue dans une zone de réaction à partir de laquelle un courant de produit contenant du carbonate d'alkylène et du catalyseur de phosphonium usé est retiré,
(b) du carbonate d'alkylène et un courant contenant du catalyseur de phosphonium usé sont séparés du courant de produit,
(c) le carbonate d'alkylène, séparé à l'étape (b) est récupéré en tant que produit,
(d) au moins une partie du courant contenant le catalyseur de phosphonium usé est soumise à une purification, pour obtenir un catalyseur de phosphonium purifié, dans lequel la partie du courant contenant le catalyseur de phosphonium usé qui est purifiée est soumise à une distillation à une température de distillation allant de 50 à 200 °C et une pression pour la distillation de 0,1 à 0,0001 bar (10 à 0,01 kPa), et
(e) le catalyseur de phosphonium purifié est recyclé vers la zone de réaction.

2. Procédé selon la revendication 1, dans lequel le catalyseur est un halogénure de phosphonium de formule R₄PHal, dans lequel Hal signifie l'halogénure et les R peuvent être identiques ou différents et peuvent être choisis parmi un groupe alkyle, alcényle, cyclique aliphatique ou aromatique.

3. Procédé selon la revendication 2, dans lequel le catalyseur est le bromure de tétra(n-butyl)phosphonium.

4. Procédé selon l'une quelconque des revendications 1 à 3, dans lequel la température de distillation vaut de 100 à 180 °C.

5. Procédé selon l'une quelconque des revendications 1 à 4, dans lequel le catalyseur de phosphonium purifié est recyclé vers la zone de réaction en présence d'un solvant.

6. Procédé selon l'une quelconque des revendications 1 à 5, dans lequel de 1 à 90 % en poids, de manière davantage préférée de 2 à 50 % en poids, de manière préférée entre toutes de 5 à 25 % en poids du courant contenant le catalyseur de phosphonium usé sont soumis à une purification.

7. Procédé selon l'une quelconque des revendications 1 à 6, dans lequel une autre partie du courant contenant le catalyseur de phosphonium usé est recyclé vers la zone de réaction.

8. Procédé selon l'une quelconque des revendications 1 à 7, dans lequel un mélange de catalyseur de phosphonium usé, de catalyseur de phosphonium purifié, d'alcool et de carbonate d'alkylène est recyclé vers la zone de réaction.

9. Procédé de préparation d'un alcane diol et de carbonate de dialkyle, comprenant la préparation d'un carbonate d'alkylène par réaction d'un oxyde d'alkylène avec du dioxyde de carbone en présence d'un catalyseur de phosphonium par le procédé selon l'une quelconque des revendications 1 à 8, la réaction d'un alcanol et du carbonate d'alkylène sur un catalyseur de transestérification, et la récupération de l'alcane diol et du carbonate de dialkyle du mélange de réaction résultant.

10. Procédé selon la revendication 9, dans lequel l'alcane diol est utilisé comme solvant en présence duquel du catalyseur de phosphonium purifié est recyclé vers la zone de réaction.
